Europäisches Patentamt

European Patent Office

Office européen des brevets

(19)

(11) Veröffentlichungsnummer: **0 081 857**
**A1**

(12) # EUROPÄISCHE PATENTANMELDUNG

(21) Anmeldenummer: 82111651.4

(22) Anmeldetag: 15.12.82

(51) Int. Cl.³: **A 61 B 17/16**

(30) Priorität: 15.12.81 DE 3149717
16.04.82 DE 3214108

(43) Veröffentlichungstag der Anmeldung: 22.06.83
Patentblatt 83/25

(84) Benannte Vertragsstaaten: CH FR GB LI SE

(71) Anmelder: Dawidowski, Gerhard, Arzbacher Strasse 2,
D-8170 Bad Tölz (DE)

(72) Erfinder: Dawidowski, Gerhard, Arzbacher Strasse 2,
D-8170 Bad Tölz (DE)

(74) Vertreter: Vogeser, Werner, Dipl.-Ing. et al,
Patentanwälte Hansmann & Vogeser
Albert-Rosshaupter-Strasse 65, D-8000 München 70 (DE)

(54) Winkelbohrlehre.

(57) Die Erfindung betrifft eine Winkelbohrlehre zum Bohren winkelig zusammenlaufender Bohrungen, insbesondere zur Durchführung chirurgischer Eingriffe am Knochen, die es auf einfache Weise ermöglicht, genaue Bohrungen definierter relativer Winkellage zu erstellen. Dies wird dadurch erreicht, daß die Bohrlehre aus einem Griff (3) mit einem Querstift (12) am proximalen Ende und winkelig gegeneinander gerichteten Führungsbuchsen (13) an den freien Enden des Querstiftes (12) besteht.

- 1 -

0081857

GERHARD DAWIDOWSKI                          Vg/BCh

Arzbacher Str. 1

D-8170 Bad Tölz


Winkelbohrlehre


Die Erfindung betrifft eine Winkelbohrlehre entsprechend dem Oberbegriff des Anspruchs 1.

Eine derartige Bohrlehre ist aus der DE-OS 29 06 054 bekannt. Diese Bohrlehre dient dazu, bei Oberschenkelhalsbrüchen einen sog. Kirschner-Draht in den Knochen einzuführen, mit dessen Hilfe dann eine Bohrung zur Aufnahme einer Schraube erstellt werden kann, mittels der die Knochenbruchstücke gegeneinander gezogen werden können. Die Bohrlehre besteht aus einem Handgriff, an dessen einem Ende eine Auflageplatte befestigt ist, und einer Führungsbuchse für den Kirschner-Draht.

Bei bestimmten chirurgischen Eingriffen an Knochen, z.B. dann, wenn sich das Gewebe in einer Gelenkpfanne gelöst hat und außerhalb der Gelenkpfanne wieder angenäht werden muß, werden von Hand schräg aufeinander zulaufende Bohrungen in den Knochen gebohrt, durch die dann mittels einer halbrunden Ahle ein Faden geführt werden kann, werden derartige Bohrungen zu tief angesetzt, wird die Gelenkpfanne beschädigt. Wird zwischen den Bohrungen nicht ein bestimmter Winkel eingehalten, kann die Ahle nicht durchgeführt werden.

Der Erfindung liegt die Aufgabe zugrunde, eine Bohrlehre zu schaffen, die es in einfacher Weise ermöglicht, genaue Bohrungen definierter relativer Winkellage zu erstellen.

Gelöst wird diese Aufgabe gemäß der Erfindung durch die im kennzeichnenden Teil des Anspruchs 1 angegebenen Merkmale. Zweckmäßige Ausgestaltungen der Erfindung ergeben sich aus den Unteransprüchen.

Beim Gebrauch wird die Bohrlehre zum Beispiel an einem Knochen aufgesetzt. In eine der beiden Führungsbuchsen wird nun ein Bohrer eingesetzt und eine Bohrung durchgeführt. Zweckmäßigerweise hat der Bohrer einen Anschlag, der die Bohrtiefe des Bohrers begrenzt. Der Bohrer wird dann aus der Führungsbuchse entfernt. In diese Führungsbuchse kann nun ein Zentrierstift eingesetzt werden, der die Bohrlehre festlegt. Mittels des Bohrers wird nun unter Zuhilfenahme der anderen Führungsbuchse eine zweite Bohrung durchgeführt, die aufgrund der definierten relativen Winkellage der Führungsbuchsen auf die zuerst erstellte Bohrung trifft. Durch die aus zwei Teilbohrungen erstellte Bohrung kann nun eine Ahle geführt werden.

Die beiden Führungsbuchsen können jeweils aus einer äußeren und einer inneren Gewindehülse bestehen, so daß der Abstand der proximalen Enden der beiden Führungsbuchsen einstellbar ist und damit unterschiedlich große Ahlen verwendet werden können.

Durch die mittels der Bohrlehre gebildeten Bohrungen soll nach dem Abnehmen der Bohrlehre mittels der Ahle ein Faden geführt werden. Dieskann jedoch zu Schwierigkeiten führen, weil die Bohrungsöffnungen nach dem Abnehmen der Bohrlehre möglicherweise nicht sofort auffindbar sind. Dies kann dadurch verhindert werden, daß in jede Führungsbuchse eine Drahtführungshülse einschiebbar ist, von denen die eine eine axiale Bohrung aufweist, die am proximalen Ende in einer senkrecht zur Hülsenlängsachse gerichteten Öffnung mündet und im Mündungsbereich gebogen verläuft, und die andere eine axiale, durchgehende Bohrung aufweist.

Nach der Bildung der beiden winkelig zusammenlaufenden Bohrungen unter Zuhilfenahme der beiden Führungsbuchsen wird in jede dieser Buchsen eine Drahtführungshülse eingesetzt. Diese Führungshülsen werden so eingesetzt, daß die Mündungsöffnung der axial verlaufenden Bohrung der einen Hülse und die seitlich mündende Öffnung der anderen Führungshülse einander gegenüberliegen. Führt man nun durch die Führungshülse, die die seitliche Öffnung aufweist, einen Draht ein, dann tritt dieser am proximalen Ende dieser Führungshülse aus und gelangt in die Mündungsöffnung der axialen Bohrung der anderen Führungshülse. Wichtig ist dabei, daß die Bohrung der einen Führungshülse im Mündungsbereich gebogen verläuft, so daß das Drehtende entsprechend verformt wird und aus der seitlichen Öffnung austritt.

Damit der Draht beim Durchschieben nicht knickt, kann die eine Führungshülse am distalen Ende eine begrenzt verschiebbare Zusatzhülse aufweisen. Beim Nachschieben des Drahtes wird diese Hülse zunächst um eine bestimmte Strecke herausgezogen, der am Hülsenende vorstehende Draht mit einer Zange erfaßt und der Draht dann zusammen mit der Zusatzhülse in die Drahtführungshülse geschoben. Auf diese Weise ist der Draht auf seiner gesamten Länge geführt und kann daher nicht knicken.

Damit die seitliche Mündungsöffnung der einen Führungshülse genau gegenüber der Mündungsöffnung der anderen Führungshülse zu liegen kommt, kann an der einen Führungshülse ein Zentrierbügel vorgesehen sein, der in eine Öffnung an der Führungsbuchse an der Winkelbohrlehre einrastbar ist. Die Führungshülse wird solange um ihre Längsachse gedreht, bis der Bügel in diese Öffnung einrastet. Die seitliche Öffnung und der Bügel haben eine solche relative Lage, daß beim Einrasten des Bügels sichergestellt ist, daß die seitliche Öffnung der Bohrungsöffnung der anderen Hülse gegenüberliegt.

Sobald der durch die eine Hülse geschobene Draht aus der anderen Hülse vorsteht, kann er mit einer Zange erfaßt und in der gewünschten Länge durchgezogen werden. Danach können die Führungshülsen abgenommen und eine Drahtschlinge oder am einen Ende eine Öse für einen durch die Bohrungen zu ziehenden Faden gebildet werden.

Die Erfindung wird nachstehend anhand der Fig. 1 bis 7 beispielsweise erläutert. Es zeigt:

Fig. 1    eine Aufsicht der Bohrlehre,

Fig. 2    eine Ansicht der Bohrlehre von unten,

Fig. 3    eine Fig. 2 entsprechende Ansicht mit eingesetztem Bohrer und Zentrierstift,

Fig. 4    eine Ausführungsform der Bohrlehre mit einstellbaren Führungsbuchsen,

Fig. 5    eine der Führungsbuchsen der Fig. 4,

Fig. 6    eine schematische Darstellung einer in eine Bohrung eingeführten Ahle.

Fig. 7    eine Aufsicht auf den proximalen Teil der
         Winkelbohrlehre in einer weiteren Auführungsform.

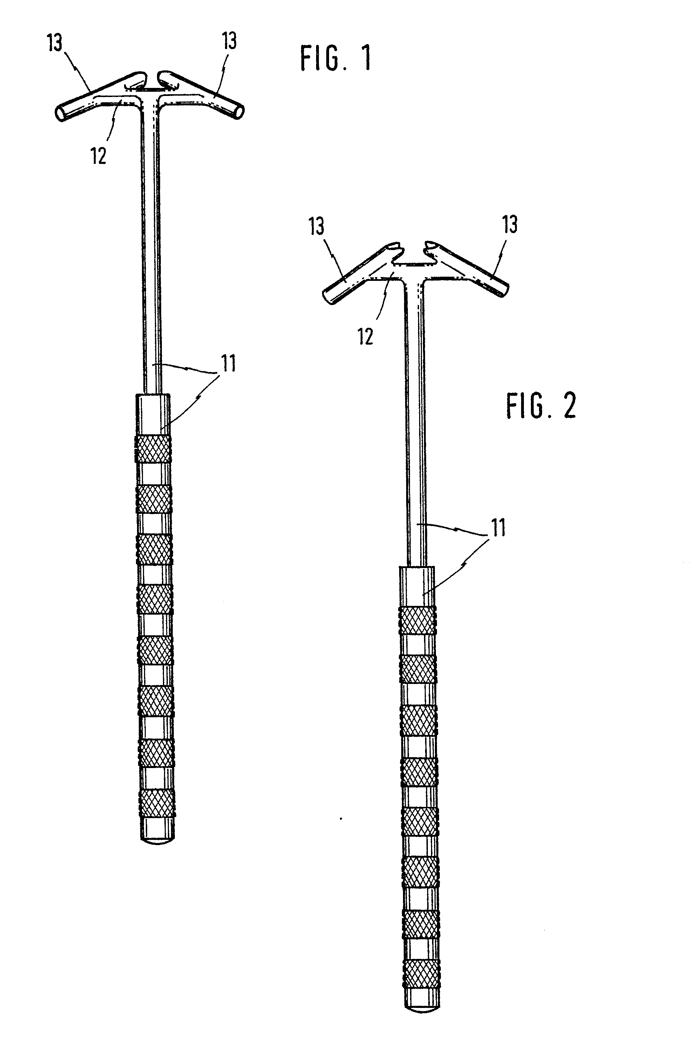

Die in den Fig. 1 bis 3 gezeigte Bohrlehre besteht
aus einem Handgriff 11, an dessen proximalen Ende ein
Quersteg 12 befestigt ist, der auch abgewinkelt sein
kann. An den freien Enden des Querstegs sind Führungsbuchsen 13 befestigt, deren proximales Ende eine schräge
Auflagefläche hat, wie insbes. Fig. 2 zeigt.

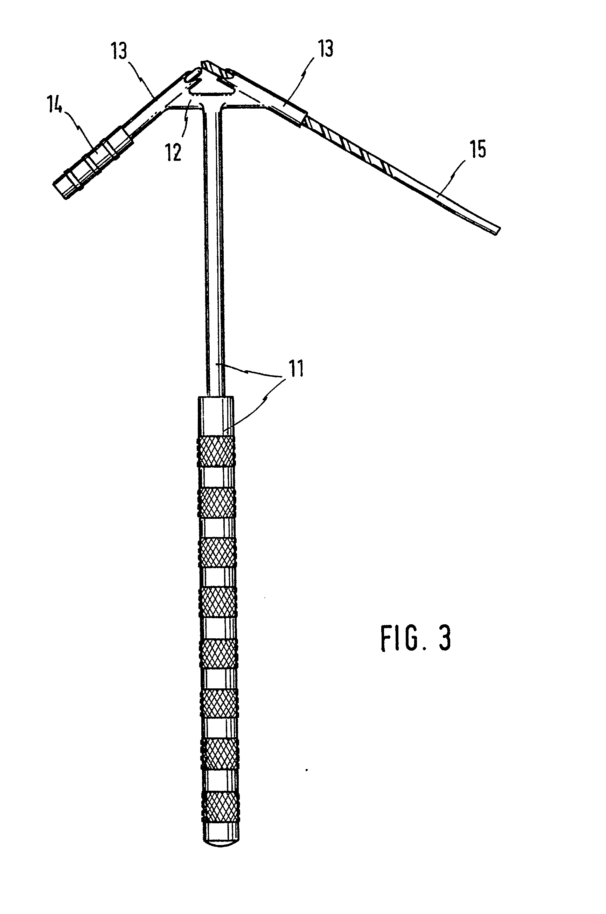

Beim Gebrauch wird die Bohrlehre am Griff 11 gehalten
und mit den schrägen Auflageflächen der Führungsbuchsen
13 z.B. auf einen Knochen aufgelegt, an dem ein Gewebe
oder ein abgerissenes Band angenäht werden soll. Durch
eine der Führungsbuchsen 13 wird ein Bohrer 15 geführt
(Fig. 3) und eine Bohrung in den Knochen gebohrt. Danach
wird in diese Führungsbuchse ein Zentrierstift 14 eingesetzt, der bis zu einem Anschlag eingeführt werden kann.
Danach wird der Bohrer 15 in die andere Führungsbuchse 13
eingeführt und eine weitere Bohrung erstellt, die auf
die zuerst erstellte trifft.

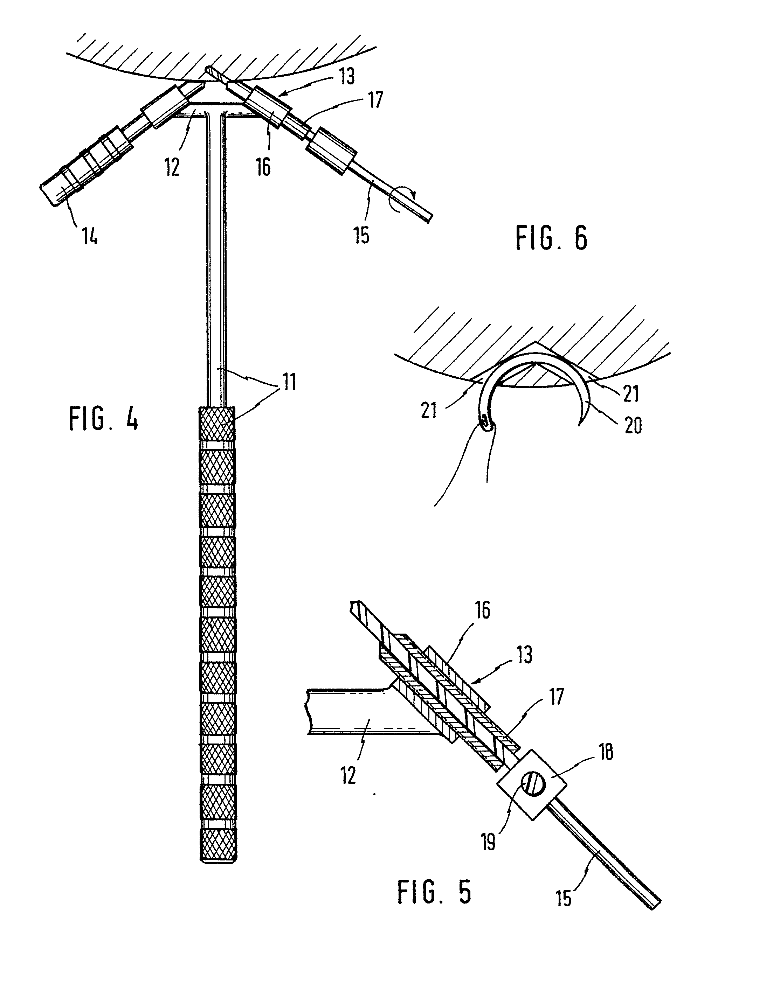

Fig. 4 zeigt eine weitere Ausführungsform der Bohrlehre,
bei der die beiden Führungsbuchsen einstellbar sind, um
die Bohrlehre an verschiedene.Ahlen anpassen zu können.
Die Führungsbuchsen bestehen, wie insbes. Fig. 5 zeigt,
aus einer äußeren Gewindehülse 16 und einer inneren
Gewindehülse 17. Die äußere Gewindehülse 16 ist am Quersteg 12 befestigt, und die innere Gewindehülse 17 kann
in der gewünschten Weise verstellt werden.

0081857

Der Bohrer 15 hat zweckmäßigerweise einen Anschlag 18, dessen Lage am Bohrer durch eine Feststellschraube 19 festlegbar ist, so daß keine zu tiefen Bohrungen auftreten können.

Fig. 6 zeigt schematisch eine mit der Bohrlehre erstellte Bohrung, die aus zwei winkelig zusammenlaufenden Teilbohrungen 21 besteht und durch die eine halbrunde Ahle 20 geführt werden kann.

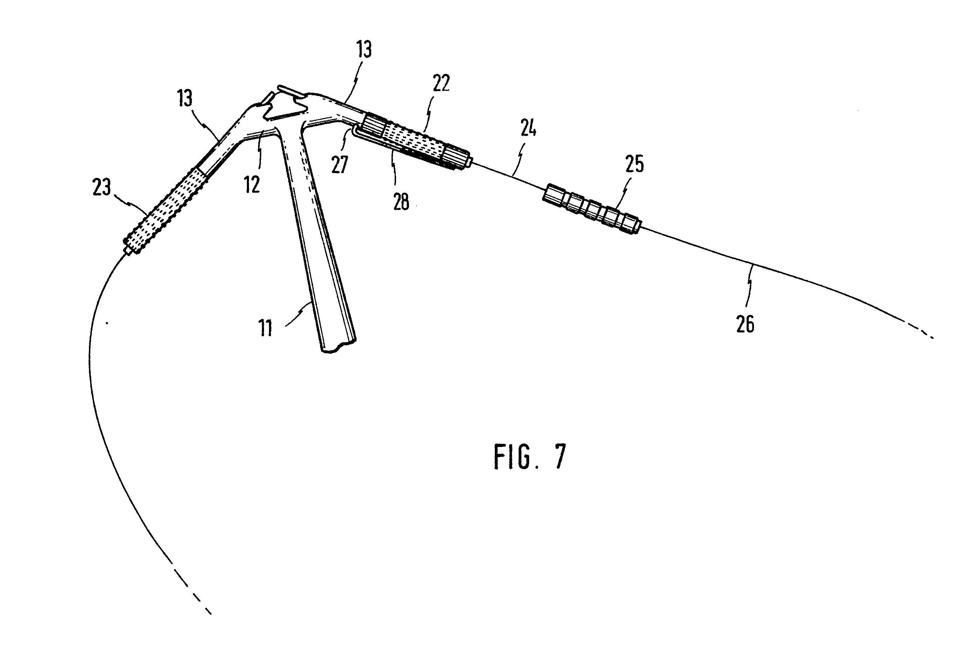

Die in Fig. 7 gezeigte Winkelbohrlehre besteht aus einem Griff 11, an dessen proximalen Ende ein Querstift 12 befestigt ist. An den freien Enden des Querstiftes sind Führungsbuchsen 13 befestigt, deren proximales Ende eine schräge Auflagefläche hat.

Beim Gebrauch wird die Bohrlehre am Griff 11 gehalten und mit den schrägen Auflageflächen der Führungsbuchsen 13 z.B. auf einen Knochen aufgelegt. Durch eine der Führungsbuchsen 13 wird ein Bohrer geführt und eine Bohrung in den Knochen gebohrt. Danach wird in diese Führungsbuchse ein Zentrierstift eingesetzt, der bis zu einem Anschlag eingeführt werden kann. Anschließend wird der Bohrer in die andere Führungsbuchse eingeführt, und es wird eine weitere Bohrung erstellt, die auf die zuerst gebildete Bohrung trifft.

Nach der Bildung der Bohrungen werden die in Fig. 7 gezeigten Drahtführungshülsen ·22 und 23 in die Führungsbuchsen 13 und die gebildeten Bohrungen geschoben. Die Führungshülse 22 eine axiale Bohrung, die am proximalen Ende in einer seitlichen Öffnung mündet.

0081857

Die Bohrung verläuft im Mündungsbereich gebogen, so daß ein durchzuschiebender Draht 26 an dieser Biegung entsprechend verformt wird und an der seitlichen Öffnung austritt.

Dieser seitlichen Öffnung gegenüber liegt die Mündungsöffnung der nur axial verlaufenden Bohrung der anderen Führungshülse 23, die in Fig. 7 etwas zurückgezogen gezeigt ist, in der Praxis jedoch so eingesetzt wird, daß beide Führungshülsen aufeinanderstoßen.

Zum Einschieben des Drahtes 26 in die Führungshülse 22 wird eine Zusatzhülse 25 verwendet, die auf einem Teil 24 der Hülse 22, der einen geringeren Durchmesser aufweist, begrenzt axial verschiebbar ist. Zum Einschieben des Drahtes wird die Zusatzhülse 25 in die in Fig. 7 gezeigte Lage zurückgeschoben, und der Draht 26 wird am distalen Ende der Zusatzhülse 25 mit einer Zange erfaßt und zusammen mit der Zusatzhülse 25 in die Hülse 22 geschoben. Dieser Vorgang wird so oft wiederholt, bis der Draht 26 aus der Hülse 23 vorsteht und dann mittels einer Zange durch beide Hülsen gezogen werden kann.

Damit beide Mündungsöffnungen der Hülsen 22 und 23 einander gegenüberliegen, hat die Hülse 22 einen Bügel 26, dessen gebogenes Ende in eine Öffnung 27 der zugehörigen Führungsbuchse 13 einrastbar ist. Sobald der Bügel 2 in die Öffnung 27 einrastet, liegt die Mündungsöffnung der Hülse 22 derjenigen der Hülse 23 gegenüber. Nach dem Abnehmen der Führungshülsen kann an einem Drahtende eine Öse zur Befestigung eines Fadens gebildet werden.

Patentansprüche

1. Winkelbohrlehre zur Durchführung chirurgischer Eingriffe an Knochen, bestehend aus einem Handgriff und einer daran befestigten Führungsbuchse, die am proximalen Ende eine Auflagefläche aufweist, d a d u r c h g e k e n n z e i c h n e t , daß zum Bohren zweier winkelig zusammenlaufender Bohrungen am proximalen Ende des Handgriffes (11) ein Quersteg (12) mit zwei winkelig gegeneinander gerichteten Führungsbuchsen (13) an dessen beiden Enden befestigt ist.

2. Bohrlehre nach Anspruch 1, d a d u r c h g e - k e n n z e i c h n e t , daß die proximalen Enden der Führungsbuchsen (13) schräge Auflageflächen haben.

3. Bohrlehre nach Anspruch 1 oder 2, g e k e n n - z e i c h n e t durch einen Zentrierstift (14), der bis zu einem Anschlag in eine der Führungsbuchsen (13) einschiebbar ist.

4. Bohrlehre nach einem der Ansprüche 1 bis 3, d a d u r c h g e k e n n z e i c h n e t , daß die

Führungsbuchsen (13) zum Einstellen des Abstandes zwischen den proximalen Enden der Führungsbuchse aus einer am Quersteg (12) befestigten äußeren Gewindebuchse (16) und einer inneren Gewindebuchse (17) bestehen.

5.    Bohrlehre nach einem der Ansprüche 1 bis 4, d a d u r c h   g e k e n n z e i c h n e t ,   daß in jede Führungsbuchse (13) eine Drahtführungshülse (22, 23) einschiebbar ist, von denen die eine (22) eine axiale Bohrung aufweist, die am proximalen Ende in einer senkrecht zur Hülsenlängsachse gerichteten Öffnung mündet und im Mündungsbereich gebogen verläuft, und die andere (23) eine axiale, durchgehende Bohrung aufweist.

6.    Bohrlehre nach Anspruch 5,   d a d u r c h   g e - k e n n z e i c h n e t ,   daß die Öffnungen der Bohrungen am proximalen Ende der Führungshülsen (22, 23) im eingeschobenen Zustand der Hülsen einander gegenüberliegen.

7.    Bohrelehre nach Anspruch 5 oder 6,   g e k e n n - z e i c h n e t   durch einen Justierbügel (26) an der einen Führungshülse (22), der an seinem freien Ende gebogen ist und mit diesem Ende in eine Öffnung (27) dieser Führungshülse einrastbar ist.

8.·    Bohrlehre nach einem der Ansprüche 5 bis 7, g e k e n n z e i c h n e t   durch eine auf der einen Führungshülse (22) axial begrenzt verschiebbare Zusatz- hülse (25).

1/4     0081857

FIG. 1

FIG. 2

FIG. 3

FIG. 4

FIG. 5

FIG. 6

0081857

FIG. 7

# 0081857

Nummer der Anmeldung

**Europäisches Patentamt**

## EUROPÄISCHER RECHERCHENBERICHT

EP 82 11 1651

| | EINSCHLÄGIGE DOKUMENTE | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| A | US-A-3 727 611 (SCHULTS) * Figuren 8-11; Spalte 4, Zeilen 18-45 * | 1,2,4 | A 61 B 17/16 |
| A | DE-C- 823 640 (BERGHÖFER) * Insgesamt * | 1 | |
| A | DE-C- 681 829 (ULRICH) | 1 | |
| A | US-A-2 200 120 (NAUTH) * Seite 2, Spalte 2, Zeile 74 - Seite 3, Spalte 1, Zeile 10; Figuren * | 1,4 | |
| D,A | DE-A-2 906 054 (SUTTER) | 1 | RECHERCHIERTE SACHGEBIETE (Int. Cl. 3) |
| P | US-A-4 335 715 (KIRKLEY) | 1 | A 61 B |
| P | EP-A-0 059 044 (ASNIS) | 1 | |

Der vorliegende Recherchenbericht wurde für alle Patentansprüche erstellt.

| Recherchenort DEN HAAG | Abschlußdatum der Recherche 04-03-1983 | Prüfer STEENBAKKER J. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument